# EUROPEAN PATENT SPECIFICATION

(11) **EP 3 344 234 B1**
(45) Date of publication and mention of the grant of the patent: **24.11.2021**
(21) Application number: 16760486.7
(22) Date of filing: 05.09.2016
(51) Int. Cl.: A61K 9/16, C08F 290/12

(54) **POLYMERS AND MICROSPHERES**
POLYMERE UND MIKROKÜGELCHEN
POLYMÈRES ET MICROSPHÈRES

(30) Priority: 03.09.2015 GB 201515602
(43) Date of publication of application: 11.07.2018
(73) Proprietor: Biocompatibles UK Limited, Farnham Surrey GU9 8QL (GB)
(72) Inventor: HEAYSMAN, Clare Louise, London Greater London EC4M 7RD (GB); LLOYD, Andrew, Brighton BN2 4GJ (GB); PHILLIPS, Gary, East Sussex BN26 6TB (GB); LEWIS, Andrew Leonard, Farnham Surrey GU8 8QL (GB)
(74) Representative: Peterreins Schley
(86) International application number: PCT/EP2016/070808
(87) International publication number: WO 2017/037276

(56) References cited:
- EP-A1- 1 810 698
- WO-A1-01/68720
- WO-A2-2007/147902

## Description

The present invention is made in the field of embolotherapy and particularly relates to the provision of charged polymers that are suitable for use in the preparation of embolic microspheres, to the microspheres themselves and to compositions comprising these.

In embolotherapy, an embolic material is delivered to the blood vessels supplying a tissue, to cause an embolisation that prevents or reduces perfusion leading to local tissue necrosis. This approach has gained popularity in the treatment of vascular tumours, particularly those of the liver, such as hepatocellular carcinoma (HCC). The embolic material is generally delivered as a solid particle, although liquid embolics are also available. Modern solid embolic materials are typically provided in the form of spherical polymeric particles, known as microspheres, which are usually provided in a range of sizes over the range 20 to 1500 microns.

In one approach, the polymer carries a charge at physiological pH, such that drugs carrying the opposite charge can be electrostatically bound to the polymer, thereby providing improved drug loading and delivery characteristics. Typical of this approach are the microspheres described in WO2004/071495 and in Jaiqui et al (1996) (Jiaqi, Y., et al. (1996). Nihon Igaku Hoshasen Gakkai Zasshi 56(1): 19-24.), which are anionically charged and are suited to the loading of cationic molecules.

It is common to provide pharmaceutical agents as salt forms, to improve their bioavailability. As salt forms, some drugs become available as anionically charged species, the provision of a positively charged embolic material offers the opportunity to load anionic species of these drugs. For example, Boudy *et al* (2002) described the loading and release of sodium indomethacin from cationically charged trisacryl ion exchange microspheres, which had been originally prepared as chromatographic media.

Embolic microspheres of trisacryl-gelatin have also been developed (Laurent *et al* 1996) and are used in the clinic as so called "bland" embolic materials. These microspheres are positively charged by virtue of their gelatin content and not as a result of a charged synthetic polymer. They are not typically used for loading and delivery of drugs due to relatively poor loading and release characteristics.

WO06027567 addresses the problem of loading and delivery of camptothecin drugs into embolic microspheres. Although the drugs are cationically charged, the specification also mentions cationic polymers in addition to anionic ones. None of these polymers where prepared and their properties were not disclosed.

Thus, although the cationically charged microspheres have been proposed for loading and delivery of drugs, there remains a need for the provision of polymers with more suitable properties, for the preparation of cationically charged embolic compositions, such as microspheres, and for the delivery of anionically charged species, such as, *i.a.* drugs, and imaging agents.

The present inventors have identified a group of polymers suitable for use in embolotherapy, which are capable of loading therapeutically useful quantities of anionically charged molecules, such as drug species and imaging agents and of delivering the drugs in a useful fashion, which have properties making them appropriate for catheter delivery and can be transformed into microspheres using simple and well understood processes.

The present invention provides a cationic microsphere comprising a polymer obtainable by crosslinking a macromer with a cationically charged vinylic co-monomer, wherein the macromer comprises 1,2 or 1,3 diol groups and pendent, cross linkable groups, and wherein the cationically charged vinylic co-monomer is a compound of the formula I 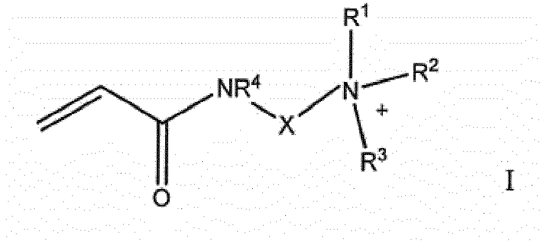 wherein X is a linear or branched C₁₋₆ alkylene, C₂₋₆ alkenylene or C₂₋₆ alkynylene group; R¹, R² and R³ are the same or different and selected from C₁₋₄ alkyl groups; R⁴ is H or C₁₋₄ alkyl, and wherein the pendant cross linkable groups are cross linked by the cationically charged vinylic co-monomer of the formula I.

The invention also provides polymeric microspheres comprising the polymer which are useful in therapy, particularly in the treatment of hypervascular tumours and in embolotherapy generally.

The polymer of the invention is water-swellable, but water insoluble; in the presence of aqueous liquid it will form a hydrogel. Polymers of this type typically comprise between 40 and 99.9% water by weight.

PVA (polyvinyl alcohol) comprises 1,3 diol groups and is one example of a suitable polymer for use in the invention. PVA polymers having a molecular weight (weight average molecular weight) of between 1000 and 500000 Daltons may be used, although those having a molecular weight of 10,000 to 100,000 are preferred.

A PVA macromer, comprises two or more ethylenically unsaturated, pendant cross linkable group per PVA polymer molecule. Preferably the PVA macromers have about 2 to 20 such groups per molecule, for instance 5 to 10 groups. These pendant groups may be vinylic or acrylic groups. Pendant acrylic groups may be provided, for instance, by reacting acrylic or methacrylic acid with PVA to form ester linkages through some of the hydroxyl groups. Methods for attaching vinylic groups capable of polymerisation, onto polyvinyl alcohol, are described in, for instance, US4,978,713, US5,508,317 and US5,583,163. The preferred macromer comprises a backbone of polyvinyl alcohol to which is linked, via a cyclic acetal linkage, an (alk)acrylaminoalkyl moiety. Example 1 of this specification describes the synthesis of such a macromer.

Preferred macromers comprise in-chain (rather than terminal) cross linkable groups such as those of the formula II, incorporating the pendant groups. 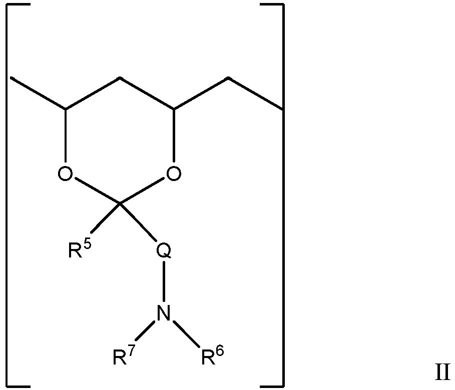 wherein
Q is a linear or branched C₁-C₈ alkylene group;
R⁵ is H, a C₁₋₆ alkyl, or a C₃₋₆ cycloalkyl;
R⁶ is an olefinically unsaturated electron attracting copolymerizable radical having
up to 25 carbon atoms; and
R⁷ is H or a C₁₋₆ alkyl.

Q is preferably a methylene, ethylene or propylene group and most preferably a methylene group.

R⁵ is preferably H or methyl, particularly H.

R⁶ is preferably a group of the formula III 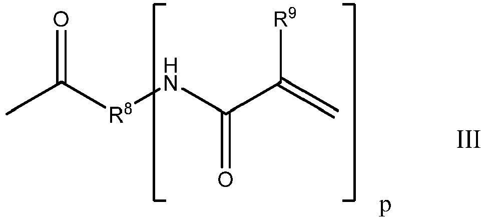 Wherein
p is 0 or 1; and
R⁹ is H or C₁₋₄ alkyl;
and wherein,
when p is 0, then R⁸ is 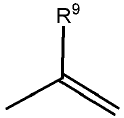
and when p is 1, R⁸ is a C₁₋₄ alkylene group.

The macromer preferably comprises cross-linkable groups of formula IIa 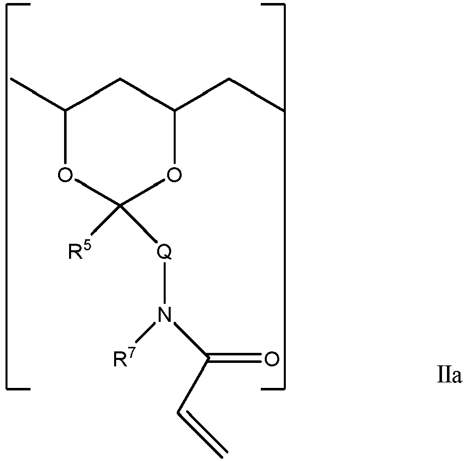 Wherein
Q is a methylene, ethylene or propylene group and most preferably a methylene group; R⁵ is H or methyl, and particularly H; and R⁷ is H or methyl, and particularly H. Thus particularly Q is a methylene group; R⁵ is H and R⁷ is H, as per formula IIb

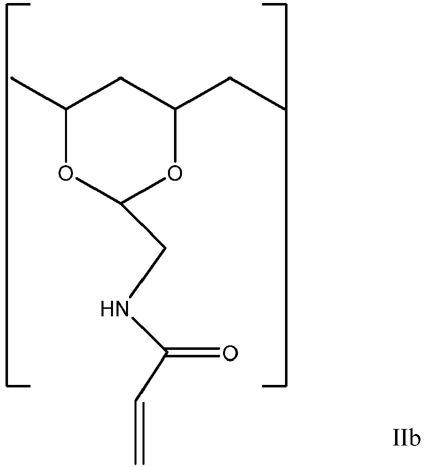

In the cationically charged vinylic monomer of the formula (I) 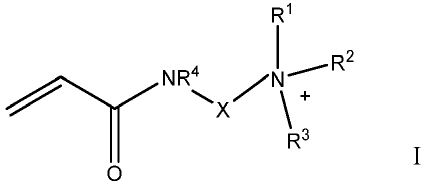 preferably
X is a linear or branched C₁₋₄ alkylene; preferably ethylene, propylene or buylene;
R¹, R² and R³ are the same or different and selected from C₁₋₄ alkyl groups; preferably methyl or ethyl
R⁴ is H or C₁₋₄ alkyl, preferably H or methyl.

Most preferably the cationically charged vinylic monomer is selected from (3-acrylamidobutyl)trimethyl ammonium salts, (3-acrylamidoethyl)trimethylammonium salts and, preferably (3-acrylamidopropyl)trimethylammonium salts. Salts are preferably chlorides.

Thus in the most preferred embodiments, the invention provides a polymer comprising groups of the formula IIa or IIb crosslinked by a cationically charged vinylic co-monomer selected from (3-acrylamidobutyl)trimethyl ammonium salts, (3-acrylamidoethyl)trimethylammonium salts and, preferably (3-acrylamidopropyl)trimethylammonium salts.

Cationically charged embolic microspheres can be produced, for example, using water in oil polymerisation techniques as previously described (e.g. WO2004/071495) and as outlined below.

The invention therefore also provides a process for the preparation of a cationic microsphere comprising providing a macromer as described above and cross linking the macromer with a cationically charged vinylic co monomer as described above. Typically a redox catalysed process is used.

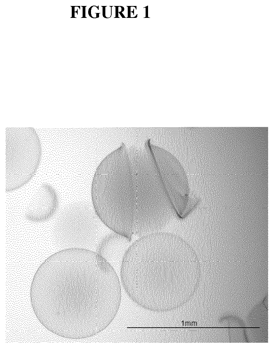

The applicants have identified that, under general process conditions, microspheres are produced, some of which have a core-shell type structure (see figure 1). The outer shell of these microspheres can rupture, particularly in aqueous preparations, and becomes detached. Such preparations are undesirable, because the small particles produced can become lodged distally from the main embolus and may lead to off target emboli and thus unpredictable embolisation. It is preferable therefore that microsphere compositions comprise no ruptured microspheres. The invention provides such compositions.

The applicants have further identified that core-shell structure and thus ruptured microspheres, can be avoided by keeping the weight% of cationic monomer between certain values. Useful polymers and particularly those of the microspheres of the present invention, comprise between 5 and 75 weight% of cationic co-monomer, preferably 10 and 70, more preferably 15 to 65 and most preferably 16 to 60 weight%. The weight % being expressed as the weight % of polymer, the remainder being macromer.

Microspheres can be separated into useful size ranges between 40 and 1500 microns, by sieving. Typically, useful size ranges are 40-70, 70-150, 100-300, 300-500, 500-700, 700-900 microns in diameter. Preferably, in sized microsphere preparations, at least 70% of microspheres are within the specified range. Preferably at least 80% or 90% and more preferably at least 95%. This results in more predictable embolization, and ease of passage down catheters, without blockage.

Due to the cross linked nature of the polymers of the present invention, the matrix is capable of allowing the passage of molecules of a broad range of molecular weights. Loading of the polymer with molecules such as drugs, is therefore not limited to low molecular weight species. Depending on the degree of cross linking, the molecular weight cut-off ranges between 40 and 250kDa. This makes the structure of the microsphere accessible to macromolecules such as peptides, proteins and nucleic acids such as DNA and RNA, as well as smaller active ingredients. By controlling the level of cationic co monomer, the molecular weight cut-off can be adjusted. Polymers having higher proportions of cationic co-monomer have a higher molecular weight cut off. Preferred MW cut-offs are in the range 40-70, 70-250 and 40-250kDa. High MW cut-off matrices allow macromolecules such as DNA, RNA and proteins to be loaded into the microspheres.

As alluded to above, the polymers and microspheres of the present invention can be loaded with pharmaceutically useful species, that may then be released within the body once the polymer or microsphere has been delivered, or alternatively, for example in the case of imaging agents, remain within the polymer in order to identify its position within the body.

The present invention therefore also provides a polymer or a microsphere as described above, comprising a pharmaceutical active or an imaging agent.

Polymers and microspheres provided by the present invention are capable of acting as carriers for a variety of molecules such as pharmaceutical actives. These molecules may be associated with the polymer in a number of ways, for example by incorporation into the polymer matrix during the process of preparing the polymer or forming the microsphere, by absorption into the polymer after formation, by precipitation within the polymer (typically limited to molecules of very low aqueous solubility e.g. less than 10g/L) (see for example WO07090897, WO07085615) or by ionic interaction. Typically actives loaded by ionic interaction will carry an anionic charge. The active preferably carries an anionic charge and is releasably bound within the polymer by ionic interactions. This allows the active to be delivered to a site within the body (for example when bound to a microsphere) and released over an extended period.

The loading of such compounds can be achieved quite readily by contacting the polymer or microspheres with solution of the compound in charged form. The loading process proceeds most advantageously in aqueous solutions, since this approach doesn't require the later removal of solvent from the preparation.

Examples below make use of model molecules to demonstrate the ability to load anionically charged species carrying varying levels of charge. It is to be noted that the range of loadable species is not limited to these model compounds.

The present invention particularly contemplates the loading of pharmaceutical actives that are anionically charged at physiological pH (7.4). Suitable species include anionically charged (acidic) drugs, oligonucleotides, DNA, RNA, anionic polypeptides, for example. Anionic imaging agents may also be loaded. Typically actives will be loaded as their charged form, such as in the form of salts (e.g. as aluminium, benzathine, calcium, ethylenediamine, lysine, meglumine, potassium, procaine, sodium, tromethamine or zinc salts). The microspheres and polymers may also be used to bind negatively charged liposomes. Suitable drugs include those having one or more carboxylate groups such as indomethacin, phenylbutazone, ketoprofen, ibuprofen, diclofenac, aspirin, warfarin, furosemide as well as sulphonamides, Particularly the microspheres and polymers may be used with anticancer drugs, such as the various carboxylate containing antifolate drugs, including methotrexate, pemetrexed, ralitrexed. pralatrexed, plevitrexed and BGC-945, which typically will be used as the salt form, e.g sodium or disodium salts.

In order to visualise polymers and particularly microspheres, within the patient, it is useful to provide polymers and microspheres that are imageable within the body, typically by incorporation of one or more imaging agents into the polymer or microsphere. These molecules may be associated with the microsphere in a number of ways, for example by incorporation into the polymer matrix during the process of forming it (e.g. as a microsphere), by absorption into the microsphere or polymer after formation, by precipitation within the microsphere or polymer (typically limited to molecules of low aqueous solubility e.g. less than 10g/L) or by ionic interaction.

Suitable imaging agents include X-ray, magnetic resonance agents, positron emission tomography (PET) agents, paramagnetic resonance agents *and so on.*

Making the microsphere X-ray imageable by making it radiopaque is one approach. A variety of methods have been proposed in the literature, to achieve this. For example Thanoo (1991) discloses a method in which barium sulphate is incorporated into the microsphere during preparation. Sodium iodide comprises an iodide ion, that can bind to the cationically charged polymer and so provide an imagable microsphere. WO2015/033093 describes a particularly convenient method of rendering the polymer radiopaque, by covalently coupling a radiopaque species to a preformed microsphere. The method involves coupling an aldehyde, comprising a covalently attached radiopaque species, such as a halogen (e.g. iodine or bromine), to preformed polymeric microspheres having 1,2 or 1,3 diol groups. The presently described polymer may comprise PVA, to which the aldehyde may conveniently be attached as described in WO2015/033093.

This latter approach results in a radiopaque polymer comprising units of the formula IV, and may also be used to render the polymers and microspheres of the present invention radiopaque.

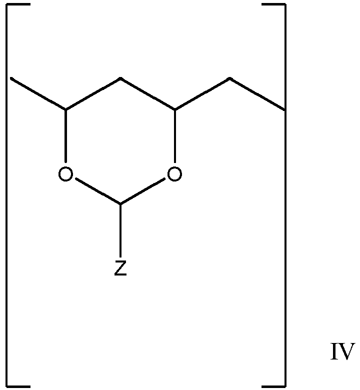

In the formula IV, Z is a group comprising one or more covalently bound radiopaque halogens, such as iodine. Particularly Z comprises a phenyl group having 1, 2, or 3 covalently bound iodines. Microspheres prepared in this manner preferably comprise at least 10% iodine by dry weight. Preferably the polymer contains at least 20% iodine by dry weight and preferably greater than 30%, 40%, 50% or 60% iodine by dry weight. A particularly useful radiopacity is obtained with polymers having between 30 and 50% iodine by dry weight.

An alternative method is to render the polymer or microsphere imageable by magnetic resonance imaging (MRI). Typically this is achieved by incorporating into the polymer or microsphere an MRI-detectable component, such as iron for example as an iron oxide particle (e.g. as described in WO09073193), or gadolinium.

In one particular embodiment, polymers and microspheres can be made imageable by positron emission tomography (PET). This approach is of particular interest because the cationically charged polymer can be charged with a negatively charged PET imageable component such as ¹⁸F ions (provided as e.g. NaF)

An alternative approach to X-ray contrast media include, *i.a.* ioxaglate, an ionic contrast agent although the microspheres can also be used with non ionic contrast agents such as iopamidol, iohexol, oxilan, iopromide and iodixanol. these compounds may be absorbed into the microspheres from aqueous solution.

The microspheres of the present invention are typically provided sterile. Sterilisation can be achieved by methods known in the art, such as autoclaving or exposure to ionising radiation. The microspheres can be provided dry (lyophilised) or as a pharmaceutical composition comprising microspheres of the invention and a pharmaceutically acceptable diluent, such as water or saline. Where they are provided dry, they are usefully provided in a sealed vial under reduced pressure (such as 0.1bar or less), such that rehydration can be achieved more rapidly (as described in WO07147902).

Suitable pharmaceutical compositions also include compositions comprising a contrast agent, in order to assist placing of the polymer or microspheres in the body. Although both ionic and non ionic contrast agents may be used, in general, non ionic contrast agents (such as, for example iopamidol, iohexol, ioxilan, ipromide and iodixanol) are preferred as they are associated with fewer adverse reactions (Katayama et al (1990) Radiology; 175:621-628) and, due to their non ionic nature, do not contribute to dissociation of any loaded drug from the polymer.

The microspheres and compositions described above may be used in a method of treatment of a patient comprising administering to the patient, cationic microspheres as described herein. The patient may be in need of therapy which comprises embolization of a blood vessel. The microspheres are typically introduced into a blood vessel and cause an embolus (embolotherapy). The approach may use microspheres that have no added active ingredient or imaging agent or they may comprise and agent as described above. Alternatively the microspheres may be administered by direct injection to a site within the body of the patient, where they act as a depot of the pharmaceutical active or imaging agent, and typically do not lead to embolization.

The blood vessel is typically one associated with a hyper vascularised tissue, such as hepatic tumours including hepatocellular carcinoma (HCC) and hepatic metastases including metastatic colorectal carcinoma (mCRC) and neuroendocrine tumours (NETs). The embolic microspheres of the invention can also be used to treat other conditions where embolisation may be effective, such as in other hypervascular conditions including uterine fibroids, prostate hyperplasia (including benign prostate hyperplasia) and for the treatment of obesity (for example by bariatric artery embolization - Weiss et al J Vase Interv Radiol. 2015 May; 26(5):613-24.) Such methods are particularly useful where a pharmaceutical active and/or imaging agent is loaded into the microspheres, and the treatment provides for the delivery of a therapeutically effective amount of the active to a patient in need thereof. The microspheres may also be used in procedures in which the microspheres are delivered to the site of action by direct injection. One approach to this is the delivery of microspheres comprising pharmaceutical actives directly to tumours or around their periphery, by injection.

### Figures

Figure 1 shows a microsphere prepared according to example 1 and having a disrupted outer layer.

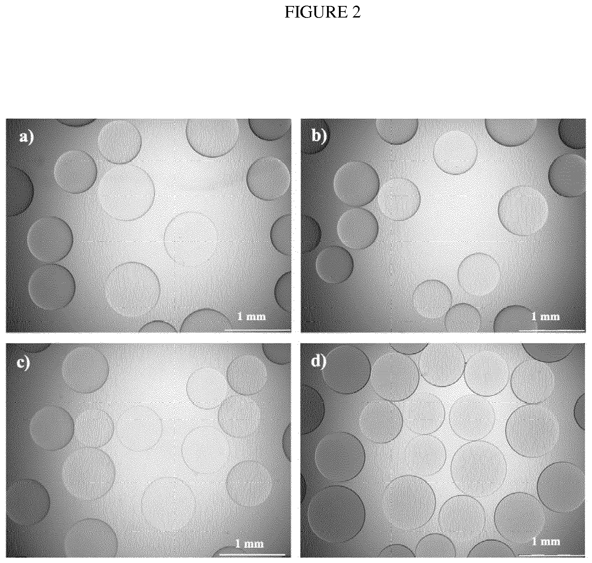

Figure 2 shows optical photomicrographs of a) APTA₁₆, b) APTA₂₇, c) APTA₄₃ and d) APTA₆₀.
Figure 3 shows Confocal Laser Scanning Microscopy images of microspheres prepared according to Example 2 after exposure to FITC-Dextrans of a variety of molecular weights.
Figure 4 gives the structures of 4 sulphonic acid dyes used as model compounds in loading and elution studies (a).1-pyrenesulfonic acid sodium salt (P1): (b) 6,8-dihydroxypyrene-1,3-disulfonic acid disodium salt (P2): (c) 8-hydroxypyrene-1,3,6-trisulfonic acid trisodium salt (P3) and (d) 1,3,6,8-pyrenetetrasulfonic acid hydrate tetrasodium salt (P4).
Figure 5 illustrates the fraction of P1, P2, P3 and P4 dyes eluted from APTA₄₃ microspheres in 200 mL of PBS (mean ± range, n=3). Initial loading was 8.6 umols per mL of microspheres.
Figure 6 illustrates the fraction of P1 eluted from APTA₁₆, APTA₄₃ and APTA₆₀ microspheres in 200 mL of PBS (average ± range, n=3). Initial loading was 8.6 umols per mL of microspheres.

### Examples

### Example 1

### (i) Synthesis of PVA macromer

Macromer may be prepared essentially according to Example 1 of WO04071495. Mowiol 8-88 PVA powder (88% hydrolised, 12% acetate content, average molecular weight about 67,000D) (150g) (Clariant, Charlotte, NC USA) is added to a 2 litre glass reaction vessel. With gentle stirring, 1000ml water is added and the stirring increased to 400rpm. To ensure complete dissolution of the PVA, the temperature is raised to 99 ±9°C for 2-3 hours. On cooling to room temperature N-acryloylaminoacetaldehyde (NAAADA) (Ciba Vision, 10 Germany) (2.49g or 0.104mmol/g of PVA) is mixed in to the PVA solution followed by the addition of concentrated hydrochloric acid (100ml). The reaction proceeds at room temperature for 6-7 hours and is then stopped by neutralisation to pH 7.4 using 2.5M NaOH.

Diafiltration is performed using a stainless steel Pellicon 2 Mini holder stacked with 0.1m² cellulose membranes having a molecular weight cut off of 3000 (Millipore Corporation, Bedford, MA USA). The macromer solution is circulated over the membranes at approximately 50psi. When the solution has been concentrated to about 1000ml the volume is kept constant by the addition of water at the same rate that the filtrate is being collected to waste until 6000ml extra has been added. Once achieved, the solution is concentrated to 20-23% solids with a viscosity of 1700-3400cP at 25°C..

### (ii) Preparation of polymer microspheres

Microspheres were synthesised in a redox catalysed reaction in a "water in oil" type system.

**Organic Phase:** 600g n-butyl acetate and 11.5g of a 10% (w/w) cellulose acetate butyrate (CAB) in ethyl acetate were added to a glass 1L jacketed vessel connected to a heater-chiller unit and stirred at approximately 300 rpm at 25°C and purged with N₂.

**Aqueous Phase:** A known amount of PVA macromer (21g non-volatile weight), 1.3g ammonium persulphate (APS), the appropriate amount of 3-acrylamidopropyl)trimethylammonium chloride (APTA) solution and an additional amount of purified water were mixed together and added to the reaction vessel. Water was added so that the total amount of water in the formulation was approximately 130g.

Polymerisation was activated through the addition of 1.6 mL TMEDA. An excess amount of N,N,N',N'-tetramethylethlenediamine (TMEDA) to APS was used to ensure complete reaction of APS. The reaction was allowed to continue for three hours at 55°C under an inert N₂ atmosphere. The microspheres were then purified by washing in ethyl acetate and acetone to remove residual CAB, before hydration and washing in water. The microspheres were heat extracted by boiling in an 80 mM disodium hydrogen phosphate in 0.29 % (w/w) NaCl solution before rehydration in water, followed by equilibration in saline.

Microspheres were produced in a range of sizes, typically between 100 to 1200 µm when hydrated in saline, and were separated into size ranges using sieves. In all formulations the total water content, weight of macromer and APS remained the same. Notation for the formulations represents the ratio of weight percentage (wt%) for APTA to macromer used in synthesis e.g. APTA₄₅ denotes 45 wt% APTA to 55 wt% macromer. Table 1 gives the weight percentage (wt%) of APTA versus macromer in example microsphere formulations.

**Table 1**

| Formulation | APTA (wt%) | Macromer (wt%) |
|---|---|---|
| APTA₀ | 0 | 100 |
| APTA₁₆ | 16 | 84 |
| APTA₂₇ | 27 | 73 |
| APTA₄₃ | 43 | 57 |
| APTA₆₀ | 60 | 40 |
| APTA₈₆ | 86 | 14 |
| APTA₁₀₀ | 100 | 0 |

Gravimetric analysis was used to determine the exact mass of polymer per volume of hydrated microspheres. A volume of microspheres fully hydrated in water, was measured out using a measuring cylinder, transferred to a vial and the water removed. The microspheres were dried under vacuum at 80 to 120 °C until a constant weight was reached. The weight of the remaining polymer was recorded and the mass per volume of microspheres determined.

Equilibrium water content measured for each of APTA₁₆, APTA₄₃ and APTA₆₀ spheres was between 98 and 99% (n=7)

### Example 2. Molecular weight cut-off of matrices.

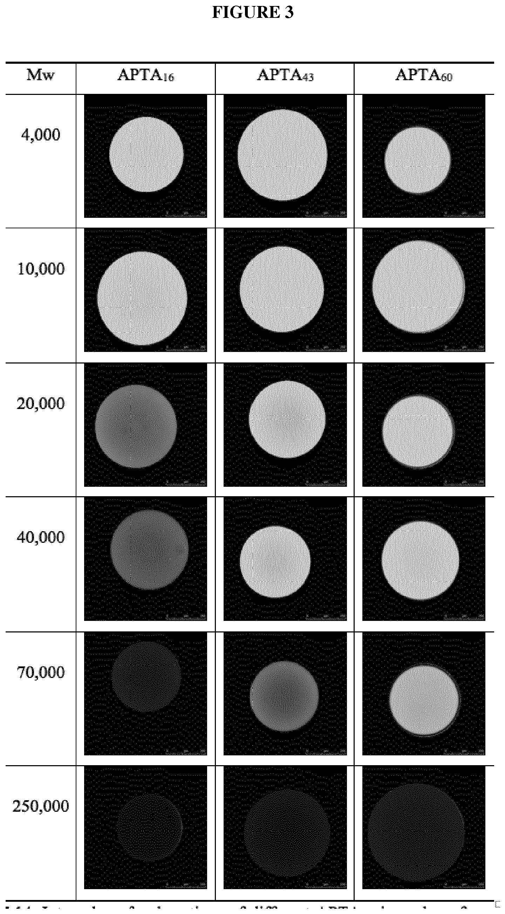

Molecular weight cut-off data was determined for each matrix formulation by exposure of microspheres, fully swollen in water, to FITC-Dextran conjugates (FITC-D) with molecular weights between 4 kDa and 250 kDa. The diffusion of FITC-Ds into the interior of the microspheres was monitored using Confocal Laser Scanning Microscopy (CLSM). Representative images of centralised regions of interest are shown in Figure 3 for APTA₁₆, APTA₄₃ and APTA₆₀. A summary of the maximum molecular weight cut-off range, above which FITC-Ds were not observed in the centre of microspheres, is presented in Table 2.

**Table 2**

| *Formulation* | *Molecular Weight Cut-Off Range (kDa)* |
|---|---|
| APTA₁₆ | 40 - 70 |
| APTA₄₃ | 70 - 250 |
| APTA₆₀ | 70 - 250 |

### Example 3: Loading of small molecules into the polymer matrix

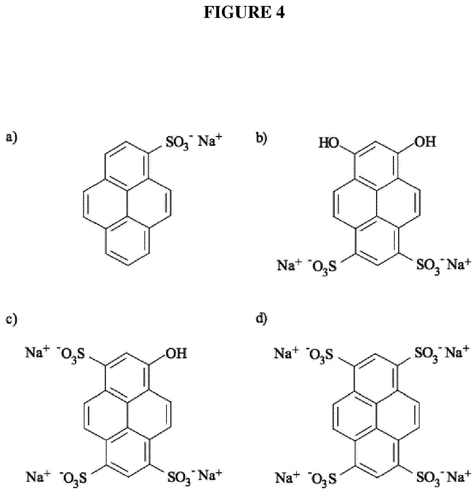

The loading and elution properties of the microspheres of the invention were characterised using a series of commercially available pyrene sulfonic acid sodium salts as model anionic drugs. The chemical structures of each dye; 1-pyrenesulfonic acid sodium salt (P1), 6,8-dihydroxypyrene-1,3-disulfonic acid disodium salt (P2), 8-hydroxypyrene-1,3,6-trisulfonic acid trisodium salt (P3) and 1,3,6,8-pyrenetetrasulfonic acid hydrate tetrasodium salt (P4) are shown in figure 4.

### (i) Loading

A measuring cylinder was used to aliquot a volume of microspheres fully hydrated in saline (e.g. 1 mL). The microspheres were then transferred to a vial and the saline solution removed. A solution of the model compound was prepared by dissolving the compound in deionised water. The solution was then added to the vial containing the slurry of microspheres. The vial was then rolled to mix at room temperature, whilst loading was monitored by removing aliquots of the loading solution.

The maximum binding capacity of each formulation was determined by mixing the microsphere slurry for 72 hours with excess test compound. The remaining solution was removed from the slurry and the microspheres were washed with water to remove residual unbound compound. The binding capacity was determined by complete elution in 500 mL of a saturated KCl solution in water mixed in 50:50 ratio with ethanol.

Samples were analysed by UV/Vis. spectrophotometry against a standard curve prepared for each compound. Maximum absorbance at 375 nm for P1, 411 nm for P2, 404 nm for P3 and 376 nm for P4 were used. Table 3 gives the loading capacity of the 4 dyes in 3 different microsphere formulations.

**Table 3.**

| Formulation | Dye | Measured bound loading capacity (mg.mL⁻¹) of microspheres |
|---|---|---|
| APTA₁₆ | **P1** | 9.1 ― 9.6 |
| | **P2** | 10.8 ― 11.2 |
| | **P3** | 6.3 ― 7.3 |
| | **P4** | 6.3 ― 6.8 |
| | | |
| APTA₄₃ | **P1** | 22.9 ― 24.1 |
| | **P2** | 22.8 ― 24.5 |
| | **P3** | 16.7 ― 18.3 |
| | **P4** | 17.1 ― 18.4 |
| | | |
| APTA₆₀ | **P1** | 31.2 ― 32.2 |
| | **P2** | 30.9 ― 33.7 |
| | **P3** | 21.2-21.5 |
| | **P4** | 22.1 ― 22.9 |

### (ii) Elution

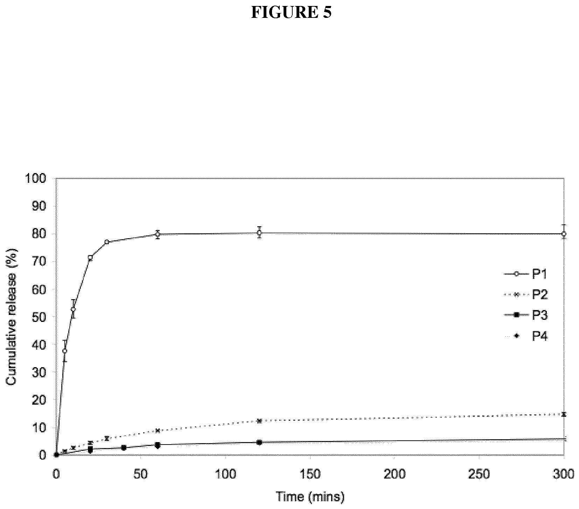

Microspheres of each polymer formulation were loaded with equal quantities of each dye. 1 ml samples of dye-loaded microspheres were added to 200 mL of PBS in an amber jar. The microsphere suspensions were rolled to provide continuous mixing. At each time point the eluent was sampled and assayed by UV/Vis spectrophotmetery as above. The volume of sampled eluent was replaced with fresh PBS to maintain the elution volume. Figure 5 illustrates the elution profiles of each dye from APTA₄₃ microspheres.

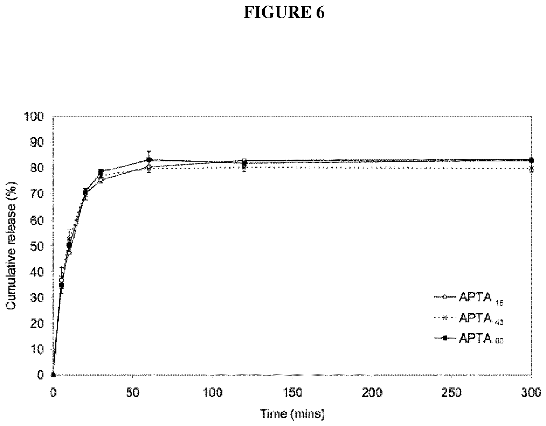

There is a difference in elution rate of the individual dyes. The monovalent dye P1 has the fastest rate of elution as 80% of the initial loaded amount was released within 60 minutes in comparison to 9% of the divalent dye P2 and approximately 3% of P3 and P4. As an illustration the elution profiles of dye P1 from APTA₁₆, APTA₄₃ and APTA₆₀, are compared in figure 6.

## Claims

1. A cationic microsphere comprising a polymer obtainable by crosslinking a macromer with a cationically charged vinylic co-monomer,
wherein the macromer comprises 1,2 or 1,3 diol groups and pendent, cross linkable groups, and
wherein the cationically charged vinylic co-monomer is a compound of the formula I 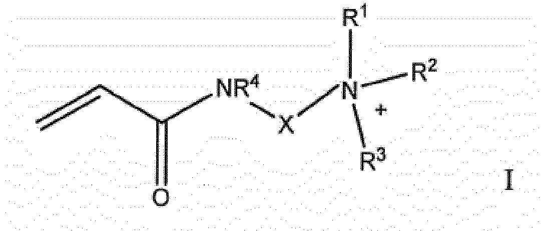 wherein
X is a linear or branched C₁₋₆ alkylene, C₂₋₆ alkenylene or C₂₋₆ alkynylene group;
R¹, R² and R³ are the same or different and selected from C₁₋₄ alkyl groups;
R⁴ is H or C₁₋₄ alkyl, and
wherein the pendant cross linkable groups are cross linked by the cationically charged vinylic co-monomer of the formula I.

2. The cationic microsphere according to claim 1 wherein the macromer comprises 1,3 diol groups.

3. The cationic microsphere according to claim 1 or 2 wherein the macromer comprises polyvinyl alcohol.

4. The cationic microsphere according to any of claims 1 to 3 wherein the macromer comprises pendant cross linkable groups of the formula: 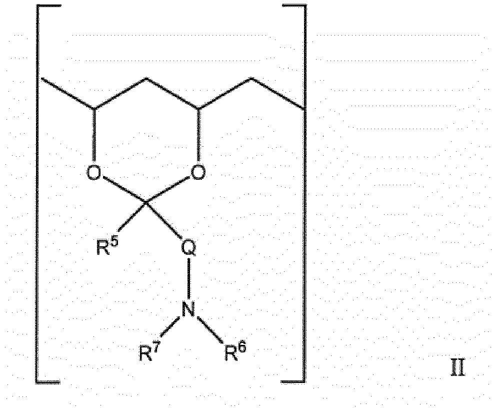 wherein
Q is a linear or branched C₁-C₈ alkylene group;
R⁵ is H, a C₁₋₆ alkyl, or a C₃₋₆ cycloalkyl;
R⁶ is an olefinically unsaturated electron attracting copolymerizable radical having up to 25 carbon atoms; and
R⁷ is H or a C₁₋₆ alkyl.

5. The cationic microsphereaccording to claim 4 wherein R⁶ is a group of the formula III 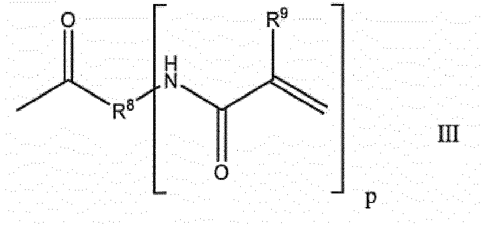 wherein
p is 0 or 1; and
R⁹ is H or C₁₋₄ alkyl;
and wherein,
when p is 0, then R⁸ is 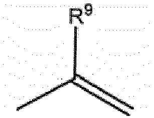
and when p is 1, R⁸ is a C₁₋₄ alkylene group.

6. The cationic microsphere according to any preceding claim, further comprising one or more iodines covalently bound to the polymer.

7. The cationic microsphere according to claim 6 comprising groups of the formula IV 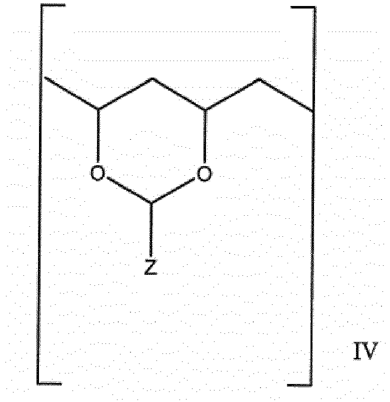 wherein Z is a group comprising one or more covalently bound iodines.

8. The cationic microsphere according to any preceding claim wherein the polymer comprises between 5 and 75 weight% of the cationically charged vinylic co-monomer.

9. The cationic microsphere according to any preceding claim comprising one or more pharmaceutical actives and/or an imaging agent.

10. The cationic microsphere according to claim 9 wherein the pharmaceutical active is bound within the polymer by ionic interactions.

11. The cationic microsphere according to claim 9 wherein the pharmaceutical active and/or imaging agent is reversibly bound within the polymer by ionic interactions.

12. A composition comprising one or more cationic microspheres according to claims 1 to 11.

13. The composition according to claim 12 comprising a pharmaceutically acceptable diluent selected from water or saline.

14. A pharmaceutical composition comprising a contrast agent and cationic microspheres according to claim 1.

15. A cationic microsphere according to claims 1 to 11 for use in a method of embolotherapy.

16. A cationic microsphere according to any of claims 1 to 11 for use in a method comprising the direct injection of the microsphere to a site within the body of a patient.

17. A sealed vessel comprising one or more sterile cationic microspheres according to claims 1 to 11 lyophilised and under a pressure of less than 0.09 MPa (0.9 bar).

## Patentansprüche

1. Kationische Mikrokugel, die ein durch Vernetzen eines Makromers mit einem kationisch geladenen vinylischen Comonomer erhältliches Polymer umfasst, wobei das Makromer 1,2- oder 1,3-Diolgruppen und seitenständige, vernetzbare Gruppen umfasst und wobei es sich bei dem kationisch geladenen vinylischen Comonomer um eine Verbindung der Formel I 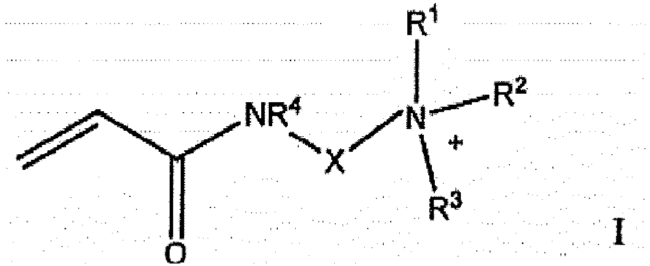 handelt, wobei
X für eine lineare oder verzweigte C₁₋₆-Alkylen-, C₂₋₆-Alkenylen- oder C₂₋₆-Alkinylengruppe steht;
R¹, R² und R³ gleich oder verschieden sind und aus C₁₋₄-Alkylgruppen ausgewählt sind;
R⁴ für H oder C₁₋₄-Alkyl steht und
wobei die seitenständigen, vernetzbaren Gruppen durch ein kationisch geladenes vinylisches Comonomer der Formel I vernetzt sind.

2. Kationische Mikrokugel nach Anspruch 1, wobei das Makromer 1,3-Diolgruppen umfasst.

3. Kationische Mikrokugel nach Anspruch 1 oder 2, wobei das Makromer Polyvinylalkohol umfasst.

4. Kationische Mikrokugel nach einem der Ansprüche 1 bis 3, wobei das Makromer seitenständige, vernetzbare Gruppen der Formel: 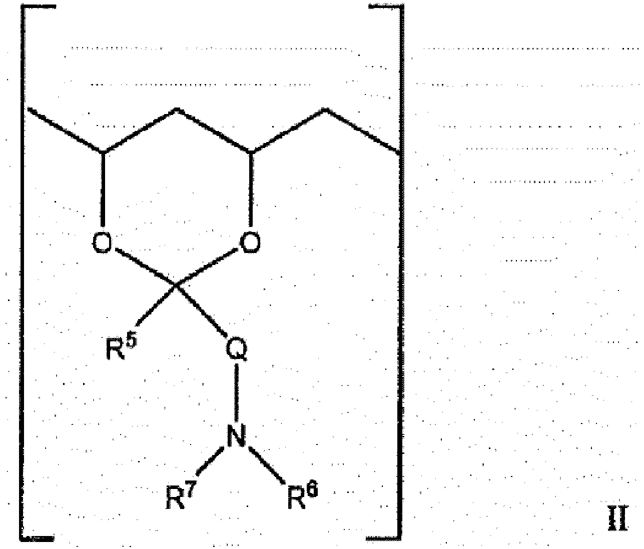 umfasst, wobei
Q für eine lineare oder verzweigte C₁₋₈-Alkylengruppe steht;
R⁵ für H, ein C₁₋₆-Alkyl oder ein C₃₋₆-Cycloalkyl steht;
R⁶ für einen olefinisch ungesättigten, Elektronen anziehenden, copolymerisierbaren Rest mit bis zu 25 Kohlenstoffatomen steht; und
R⁷ für H oder ein C₁₋₆-Alkyl steht.

5. Kationische Mikrokugel nach Anspruch 4, wobei R⁶ für eine Gruppe der Formel III 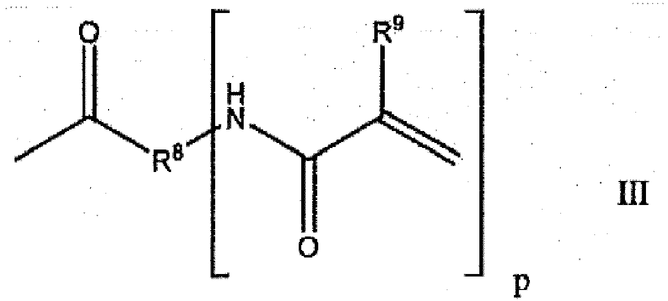 steht, wobei
p 0 oder 1 ist; und
R⁹ für H oder C₁₋₄-Alkyl steht; und wobei dann,
wenn p 0 ist, R⁸ für 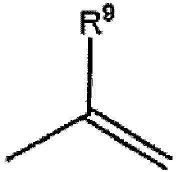 steht,
und, wenn p 1 ist, R⁸ für eine C₁₋₄-Alkylengruppe steht.

6. Kationische Mikrokugel nach einem der vorhergehenden Ansprüche, die ferner ein oder mehrere kovalent an das Polymer gebundene Iod-Atome umfasst.

7. Kationische Mikrokugel nach Anspruch 6, die Gruppen der Formel IV 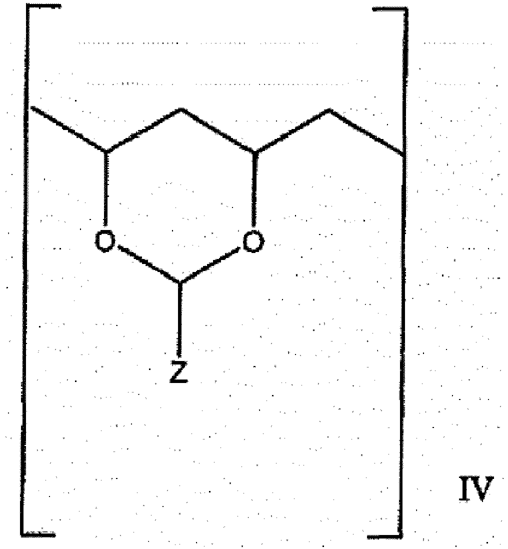 umfasst, wobei Z für eine Gruppe steht, die ein oder mehrere kovalent gebundene Iod-Atome umfasst.

8. Kationische Mikrokugel nach einem der vorhergehenden Ansprüche, wobei das Polymer zwischen 5 und 75 Gewichts-% des kationisch geladenen vinylischen Comonomers umfasst.

9. Kationische Mikrokugel nach einem der vorhergehenden Ansprüche, die ein oder mehrere pharmazeutische Wirkstoffe und/oder ein Bildgebungsmittel umfasst.

10. Kationische Mikrokugel nach Anspruch 9, wobei der pharmazeutische Wirkstoff in dem Polymer durch ionische Wechselwirkungen gebunden ist.

11. Kationische Mikrokugel nach Anspruch 9, wobei der pharmazeutische Wirkstoff und/oder das Bildgebungsmittel in dem Polymer reversibel durch ionische Wechselwirkungen gebunden ist.

12. Zusammensetzung, die ein oder mehrere kationische Mikrokugeln nach den Ansprüchen 1 bis 11 umfasst.

13. Zusammensetzung nach Anspruch 12, die ein aus Wasser und Kochsalzlösung ausgewähltes pharmazeutisch unbedenkliches Verdünnungsmittel umfasst.

14. Pharmazeutische Zusammensetzung, umfassend ein Kontrastmittel und Kationische Mikrokugeln nach Anspruch 1.

15. Kationische Mikrokugel nach den Ansprüchen 1 bis 11 zur Verwendung bei einem Verfahren der Embolotherapie.

16. Kationische Mikrokugel nach einem der Ansprüche 1 bis 11 zur Verwendung bei einem Verfahren, das die direkte Injektion der Mikrokugel an eine Stelle im Körper eines Patienten umfasst.

17. Verschlossenes Behältnis, das ein oder mehrere sterile kationische Mikrokugeln nach den Ansprüchen 1 bis 11, die lyophilisiert sind und unter einem Druck von weniger als 0,09 MPa (0,9 bar) stehen, umfasst.

## Revendications

1. Microsphère cationique comprenant un polymère qui peut être obtenu par réticulation d'un macromère avec un co-monomère vinylique chargé de manière cationique,
le macromère comprenant des groupes 1,2-diol ou 1,3-diol et des groupes pendants, réticulables, et
le co-monomère vinylique chargé de manière cationique étant un composé de la formule I 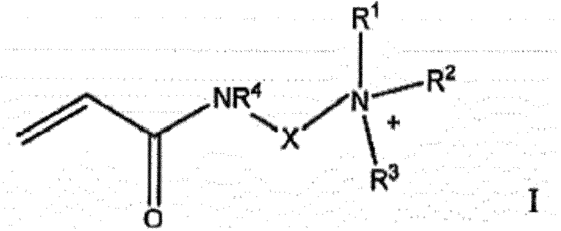
X étant un groupe C₁₋₆ alkylène, C₂₋₆ alcénylène ou C₂₋₆ alcynylène, linéaire ou ramifié ;
R¹, R² et R³ étant identiques ou différents et choisis parmi des groupes C₁₋₄ alkyle ;
R⁴ étant H ou C₁₋₄ alkyle, et
les groupes pendants réticulables étant réticulés par le co-monomère vinylique chargé de manière cationique de la formule I.

2. Microsphère cationique selon la revendication 1, le macromère comprenant des groupes 1,3-diol.

3. Microsphère cationique selon la revendication 1 ou 2, le macromère comprenant un poly(alcool vinylique).

4. Microsphère cationique selon l'une quelconque des revendications 1 à 3, le macromère comprenant des groupes pendants réticulables de la formule : 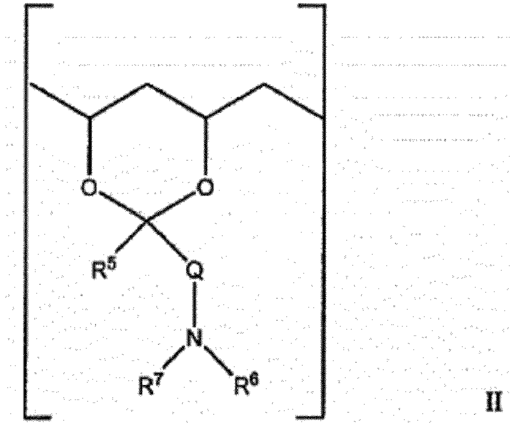
Q étant un groupe C₁₋₈ alkylène linéaire ou ramifié ;
R⁵ étant H, un C₁₋₆ alkyle, ou un C₃₋₆ cycloalkyle ;
R⁶ étant un radical copolymérisable attracteur d'électrons oléfiniquement insaturé possédant jusqu'à 25 atomes de carbone ; et
R⁷ étant H ou un C₁₋₆ alkyle.

5. Microsphère cationique selon la revendication 4, R⁶ étant un groupe de la formule III 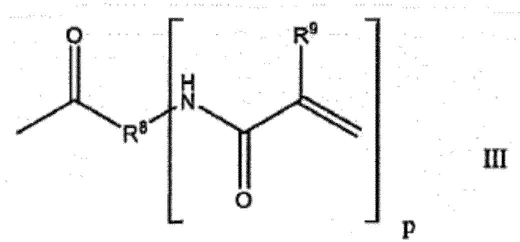
p étant 0 ou 1 ; et
R⁹ étant H ou C₁₋₄ alkyle ; et,
lorsque p est 0, alors R⁸ est 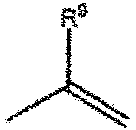
et lorsque p est 1, R⁸ est un groupe C₁₋₄ alkylène.

6. Microsphère cationique selon une quelconque revendication précédente, comprenant en outre un ou plusieurs iodes liés de manière covalente au polymère.

7. Microsphère cationique selon la revendication 6 comprenant des groupes de la formule IV 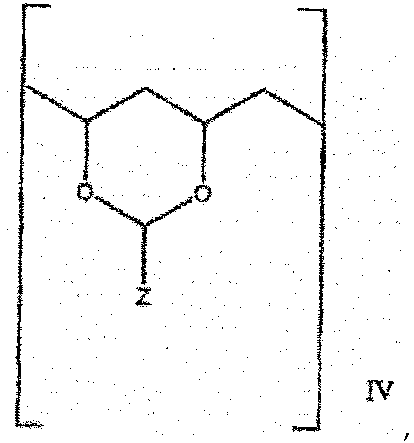 Z étant un groupe comprenant un ou plusieurs iodes liés de manière covalente.

8. Microsphère cationique selon une quelconque revendication précédente, le polymère comprenant entre 5 et 75 % en poids du co-monomère vinylique chargé de manière cationique.

9. Microsphère cationique selon une quelconque revendication précédente comprenant un ou plusieurs agents actifs pharmaceutiques et/ou un agent d'imagerie.

10. Microsphère cationique selon la revendication 9, l'agent actif pharmaceutique étant lié au sein du polymère par des interactions ioniques.

11. Microsphère cationique selon la revendication 9, l'agent actif pharmaceutique et/ou l'agent d'imagerie étant lié(s) de manière réversible au sein du polymère par des interactions ioniques.

12. Composition comprenant une ou plusieurs microsphères cationiques selon les revendications 1 à 11.

13. Composition selon la revendication 12 comprenant un diluant pharmaceutiquement acceptable choisi parmi l'eau et une solution saline.

14. Composition pharmaceutique comprenant un agent de contraste et des microsphères cationiques selon la revendication 1.

15. Microsphère cationique selon les revendications 1 à 11 pour une utilisation dans un procédé d'embolothérapie.

16. Microsphère cationique selon l'une quelconque des revendications 1 à 11 pour une utilisation dans un procédé comprenant l'injection directe de la microsphère à un endroit dans le corps d'un patient.

17. Récipient scellé comprenant une ou plusieurs microsphères cationiques stériles selon les revendications 1 à 11 lyophilisées et sous une pression inférieure à 0,09 MPa (0,9 bar).
